(19) Europäisches Patentamt
European Patent Office
Office européen des brevets

(11) Veröffentlichungsnummer: **0 007 058**
**B1**

(12) # EUROPÄISCHE PATENTSCHRIFT

(45) Veröffentlichungstag der Patentschrift: **30.12.81**

(51) Int. Cl.³: **G 01 N 33/92**, C 12 Q 1/32

(21) Anmeldenummer: **79102246.0**

(22) Anmeldetag: **03.07.79**

(54) **Verfahren und Reagens zur Bestimmung von Glycerin.**

(30) Priorität: **18.07.78 DE 2831580**

(43) Veröffentlichungstag der Anmeldung: **23.01.80 Patentblatt 80/2**

(45) Bekanntmachung des Hinweises auf die Patenterteilung: **30.12.81 Patentblatt 81/52**

(84) Benannte Vertragsstaaten: **BE CH DE FR GB IT LU NL SE**

(56) Entgegenhaltungen:
**DE-A1-2 512 266**
**DE-A1-2 512 267**
**DE-A1-2 742 699**

**Chemical Abstracts Band 60, Nr. 4, 17. Februar 1964 (COLUMBUS, OHIO, USA)**
**F.G. WINDER et al.: "Effects of iron deficiency and of zinc deficiency on the activities of some enzymes in Mycobacterium smegmatis"**
**Spalte 4490F**

**Chemical Abstracts Band 55, Nr. 25, 11. Dezember 1961 (COLUMBUS, OHIO, USA)**
**F.G. WINDER et al.: "Levels of some enzymes in normal, in iron-deficient and in zinc-deficient Mycobacterium smegmatis"**
**Spalte 26 102F**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Möllering, Hans, Herrestrasse 10, D-8132 Tutzing (DE)**
Erfinder: **Looser, Siegfried, Dr., Giesinger Strasse 3, D-8120 Weilheim (DE)**

(74) Vertreter: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

EP 0 007 058 B1

Verfahren und Reagens zur Bestimmung von Glycerin.

Die Erfindung betrifft ein Verfahren und ein Reagens zur Bestimmung von Glycerin.

Bekannt ist ein Verfahren zur Bestimmung von Glycerin mit Glycerindehydrogenase, Diaphorase als Elektronenüberträger, Nicotinamid-adenosindinucleotid (NAD) und Tetrazoliumsalz. Dabei wird ein Farbstoff gebildet, der sich je nach dem verwendeten Tetrazoliumsalz im sichtbaren oder im ultravioletten Licht leicht bestimmen lässt (DE-Offenlegungsschrift 24 59 087).

Ein Nachteil dieses Tests ist ein verzögertes Anlaufen der Reaktion. Dies ist auf die Glycerindehydrogenase zurückzuführen, die längere Zeit braucht, bis sie ihre maximale Reaktionsgeschwindigkeit erreicht. Ausserdem zeigt es sich, dass auch bei Verwendung des gleichen Ausgangsmaterials für die Gewinnung der Glycerindehydrogenase diese hinsichtlich ihrer Reaktionsgeschwindigkeit noch erheblichen Schwankungen unterliegt. Bei der Standardisierung dieses Tests muss daher aus Sicherheitsgründen eine relativ lange Messdauer vorgeschrieben werden, um sicherzugehen, dass die maximale Reaktionsgeschwindigkeit auch erreicht wird. Aufgabe der Erfindung ist es, diesen Nachteil zu beseitigen.

Gelöst wird diese Aufgabe erfindungsgemäss durch ein Verfahren zur Bestimmung von Glycerin mit Glycerindehydrogenase, einen Elektronenüberträger, NAD und Tetrazoliumsalz, welches dadurch gekennzeichnet ist, dass man wenigstens 1 µg Zink in Form eines Zinksalzes je 50 U Glycerindehydrogenase zusetzt.

Der Erfindung liegt die überraschende Feststellung zugrunde, dass Zinkionen in ganz bestimmten Konzentrationen die Glycerindehydrogenase derart zu aktivieren vermögen, dass ihre maximale Reaktionsgeschwindigkeit sehr rasch erreicht wird. Die maximale Reaktionsgeschwindigkeit selbst wird nicht verändert. Der erfindungsgemässe Befund ist überraschend,da es aus J. Biol. Chem. 235, Nr. 6, 1820 (1960) bekannt war, dass Zinkionen die Glycerindehydrogenase inhibieren. So wurde für eine Zinkkonzentration von $2{,}1 \times 10^{-5}$ M bereits eine 50 %ige Inhibierung beschrieben.

Als Zinksalz wird vorzugsweise $ZnCl_2$ verwendet. Es lassen sich jedoch andere Zinksalze, die gut wasserlöslich sind und deren Anion keinen schädlichen Einfluss auf die Reaktion ausübt, in gleicher Weise verwenden. Typische Beispiele für andere geeignete Zinksalze sind Zinksulfat, Zinkacetat, und ähnliche.

Die besten Ergebnisse erhält man bei einer Konzentration zwischen 0,01 und 1,0 mMol/l Zinksalz im Testansatz. Bei Überschreiten dieser oberen Konzentrationsgrenze tritt zwar immer noch die Aktivierung ein, es macht sich jedoch zunehmend eine Proteindenaturierung und damit Auftreten einer Trübung bemerkbar.

Das erfindungsgemässe Verfahren eignet sich sowohl zur Bestimmung von Glycerin in biologischen Flüssigkeiten oder anderem Material als auch zum Einsatz im Rahmen von Bestimmungen von Glycerinderivaten, in deren Verlauf Glycerin freigesetzt wird. Insbesondere ist das Verfahren wichtig für die Bestimmung von Triglyceriden, wobei die Triglyceridverseifung vorzugsweise enzymatisch erfolgt, aber auch alkalisch durchgeführt werden kann. Ein anderes Beispiel für eine kombinierte Bestimmung, in deren Rahmen das erfindungsgemässe Verfahren eingesetzt werden kann, ist die Phospholipidbestimmung.

Weitere Einsatzmöglichkeiten für das Verfahren sind die Bestimmung von Glycerinäthern und Glycerinestern, z.B. Apfelsäureglycerinester und Zitronensäureglycerinester, die als Geschmacks- und Geruchssubstanzen Verwendung finden; weiterhin Glycerindiester und Glycerintriester, die in Form von Acetin (Glycerintriacetat) und Butyrin (Glycerintributyrat) ebenfalls als Geruchsstoffe Verwendung finden.

Die erfindungsgemässe Umsetzung beruht auf folgenden Reaktionsgleichungen:

1) $\text{Glycerin} + \text{NAD}^+ \xrightarrow{\text{Glycerin-DH}} \text{Dihydroxyaceton} + \text{NADH} + \text{H}^+$

2) $\text{NADH} + \text{H}^+ + \text{Tetrazoliumsalz} \xrightarrow{\text{Diaphorase}} \text{Formazan (gefärbt)} + \text{NAD}^+$

Als Elektronenüberträger wird Diaphorase bevorzugt. Beispiele für andere geeignete Elektronenüberträger sind Phenazinmethosulfat (PMS), 8-Dimethylamino-2,3-benzophenoxazin (Meldolablau) und 1-Methoxy-5-methyl-phenazinium-methylsulfat. Andere Elektronenüberträger für Tetrazoliumsalze sind dem Fachmann bekannt.

Im Rahmen des erfindungsgemässen Verfahrens lassen sich zahlreiche Tetrazoliumsalze verwenden. Diese sind dem Fachmann bekannt und brauchen hier nicht weiter beschrieben zu werden. Typische Beispiele für geeignete Tetrazoliumsalze sind Nitroblautetrazoliumchlorid (NBT), 3-(4',5'-Dimethyl-thiazolyl-2)-2,4-diphenyl-tetrazoliumbromid (MTT), 2-(p-Jodophenyl)-3-(p-nitrophenyl)-5-phenyl-tetrazoliumchlorid (INT), 2,2',5,5'-Tetra-(p-nitrophenyl)-3,3'-(3-dimethoxy-4-diphenylen)-ditetrazoliumchlorid (TNBT), 2,2,5-Triphenyltetrazoliumchlorid (TT) und Neotetrazoliumchlorid (NT).

Ein weiterer Gegenstand der Erfindung ist ein Reagens zur Bestimmung von Glycerin auf Basis von Glycerindehydrogenase, einem Elektronenüberträger, NAD, Tetrazoliumsalz und Puffer,

welches dadurch gekennzeichnet ist, dass es wenigstens 1 µg Zink in Form eines Zinksalzes je 50 U Glycerindehydrogenase enthält.

In einer bevorzugten Ausführungsform enthält ein derartiges Reagens
5 bis 20 U/ml Glycerindehydrogenase,
0,2 bis 1,5 U/ml Diaphorase,
0,5 bis 5 mMol/l NAD,
0,1 bis 0,5 mMol/l Tetrazoliumsalz,
0,05 bis 0,2 Mol/l Puffer, pH 7,5 bis 9,0,
0 bis 0,5% Detergenz und
0,01 bis 1,0 mMol/l Zinksalz.

Das obige Reagens kann in Form einer fertigen wässrigen Lösung oder in Form einer trockenen Vormischung vorliegen, die zum Auflösen in Wasser bzw. Pufferlösung bestimmt ist und dann die obigen Konzentrationen ergibt. Ausser den aufgeführten Substanzen kann das Reagens zusätzlich noch Stabilisatoren, wie Ammoniumsulfat, Cholsäure und ähnliche Zusätze, die dem Fachmann bekannt sind, enthalten. Als Puffer wird Glycylglycinpuffer bevorzugt, besonders bevorzugt ist 0,1-molarer Glycylglycin-puffer, pH 8,0 bis 8,2. Die Diaphorase kann durch eine entsprechende Menge eines anderen Elektronenüberträgers ersetzt werden.

Bei Verwendung von Nitroblau-tetrazoliumchlorid (NBT) als Tetrazoliumsalz erfolgt die Messung zweckmässig bei 546 nm.

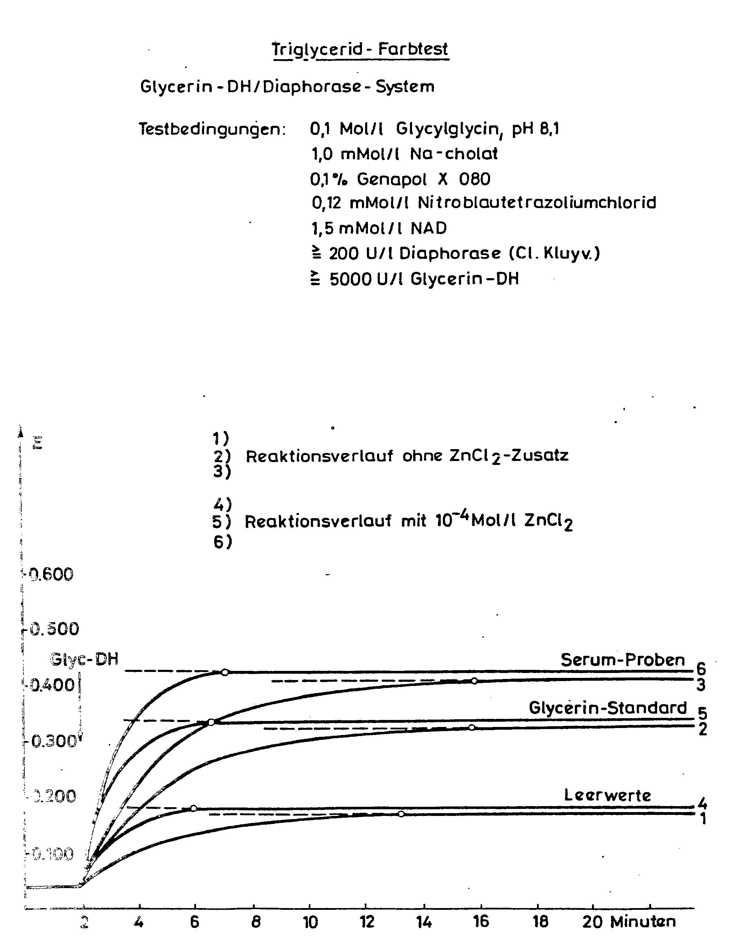

Die durch das erfindungsgemässe Verfahren erzielte Verkürzung der für die Glycerinbestimmung erforderlichen Zeit geht aus der beigefügten Fig. 1 der Zeichnung hervor. Sie zeigt den Reaktionsverlauf, dargestellt in gemessener Extinktion bei 546 nm gegen die Zeit aufgetragen. Die Kurven 1, 2 und 3 geben den Reaktionsverlauf ohne Zinkzusatz wieder, die Kurven 4, 5 und 6 den Reaktionsverlauf bei Zusatz von $10^{1-4}$ Mol/l $ZnCl_2$.

Die Testbedingungen waren wie folgt:

0,1 Mol/l Glycylglycin-puffer, pH 8,1
1,0 mMol/l Natriumchlorat,
0,1% Detergenz,
0,12 mMol/l NBT,
1,5 mMol/l NAD,
200 U/l Diaphorase,
5000 U/l Glycerindehydrogenase.

Der Start der Reaktion erfolgte nach 2 Minuten durch den Zusatz der Glycerindehydrogenase. Man erkennt, dass erfindungsgemäss die maximale und konstante Reaktionsgeschwindigkeit nach 4 bis 5 Minuten erreicht wird, während ohne den Zinkzusatz erst nach etwa 14 Minuten konstante Werte erreicht werden. Durch die Erfindung wird daher die erforderliche Reaktionszeit auf etwa ⅓ gesenkt, was insbesondere beim Einsatz auf Analysenautomaten einer Verdreifachung der Analysenkapazität gleichkommt.

Die folgenden Beispiele erläutern die Erfindung weiter.

Beispiel 1
Kombinierte Bestimmung der Triglyceride durch Spaltung und Messung des freigesetzten Glycerins.

Es wird ein Reagens folgender Zusammensetzung verwendet:

| | |
|---|---|
| Glycylglycin | 0,1 Mol/l |
| pH | 8,1 |
| Ammonsulfat | 100 mMol/l |
| Cholsäure | 1,0 mMol/l |
| Zinkchlorid | 0,1 mMol/l |
| Detergenz | 0,1% |
| NAD | 1,5 mMol/l |
| Tetrazoliumsalz | 0,12 mMol/l |
| Lipase | 1,0 U/ml |
| Glycerindehydrogenase | 15 U/ml |
| Diaphorase | 0,5 U/ml |

Die Durchführung der Bestimmung geschieht wie folgt:

In Reagenzgläser pipettieren:

| | Leerwert | Standard | Probe |
|---|---|---|---|
| Reagenslösung | 2,0 ml | 2,0 ml | 2,0 ml |
| Standard | — | 0,02 ml | — |
| Serum | — | — | 0,02 ml |

mischen, 15 Minuten bei 20 bis 25°C inkubieren. Danach innerhalb weiterer 15 Minuten Extinktion der Probe und Extinktion des Standards gegen Leerwert messen. Inkubationstemperatur: 20 bis 25°C; Messwellenlänge: Hg 546 nm.

Die Berechnung des Ergebnisses erfolgt nach folgender Formel:

$$c_{Pr} = \frac{\triangle E_{Pr}}{\triangle E_{St}} \times c_{St}$$

**Patentansprüche**

1. Verfahren zur Bestimmung von Glycerin mit Glycerindehydrogenase, Elektronenüberträger, NAD und Tetrazoliumsalz, dadurch gekennzeichnet, dass man wenigstens 1 µg Zink in Form eines Zinksalzes je 50 U Glycerindehydrogenase zusetzt.

2. Verfahren nach Anspruch 1, dadurch gekennzeichnet, dass man als Zinksalz $ZnCl_2$ verwendet.

3. Reagens zur Bestimmung von Glycerin auf Basis von Glycerindehydrogenase, Elektronenüberträger, NAD, Tetrazoliumsalz und Puffer, dadurch gekennzeichnet, dass es wenigstens 1 µg Zink in Form eines Zinksalzes je 50 U Glycerindehydrogenase enthält.

4. Reagens nach Anspruch 3, enthaltend
5 bis 20 U/ml Glycerindehydrogenase,
0,2 bis 1,5 U/ml Diaphorase,
0,5 bis 5 mMol/l NAD,
0,1 bis 0,5 mMol/l Tetrazoliumsalz,
0,05 bis 0,2 Mol/l Puffer, pH 7,5 bis 9,5,
0 bis 0,5% Detergenz und
0,01 bis 1,0 mMol/l Zinksalz.

## Claims

1. Process for the determination of glycerin by glycerin dehydrogenase, electron transmitter, NAD and tetrazolium salt, whereby at least 1 µg of zinc is added in the form of a zinc salt per 50 U of glycerin dehydrogenase.

2. Process according to claim 1, whereby $ZnCl_2$ is used as the zinc salt.

3. Reagent for the determination of glycerin on the basis of glycerin dehydrogenase, electron transmitter, NAD, tetrazolium salt and buffer, whereby it contains at least 1 µg of zinc in the form of a zinc salt per 50 U glycerin dehydrogenase.

4. Reagent according to claim 3, containing
5 to 20 U/ml glycerin dehydrogenase,
0.2 to 1.5 U/ml diaphorase,
0.5 to 5 mMol/l NAD,
0.1 to 0.5 mMol/l tetrazolium salt
0.05 to 0.2 Mol/l buffer, pH 7.5 to 9.5,
0 to 0.5% detergent and
0.01 to 1.0 mMol/l zinc salt

## Revendications

1. Procédé pour le dosage du glycérol au moyen de glycéroldéshydrogenase, d'agent de transfert d'électrons, de NAD et de sel de tétrazolium, caractérisé en ce qu'on ajoute au moins 1 µg de zinc sous forme d'un sel de zinc pour 50 U de glycéroldéshydrogénase.

2. Procédé selon la revendication 1, caractérisé en ce qu'on utilise $ZnCl_2$ en tant que sel de zinc.

3. Réactif pour le dosage du glycérol a base de glycéroldéshydrogénase, d'agent de transfert d'électrons, de NAD, de sel de tétrazolium et de tampon, caractérisé en ce qu'il contient au moins 1 µg de zinc sous forme d'un sel de zinc pour 50 U de glycéroldéshydrogénase.

4. Réactif selon la revendication 3, contenant
5 à 20 U/ml de glycéroldéshydrogénase,
0,2 à 1,5 U/ml de diaphorase,
0,5 à 5 mmoles/l de NAD,
0,1 à 0,5 mmole/l de sel de tétrazolium,
0,05 à 0,2 mole/l de tampon, pH 7,5 à 9,5,
0 à 0,5% de détergent et
0,01 à 1,0 mmole/l de sel de zinc.

# Triglycerid - Farbtest

Glycerin - DH/Diaphorase - System

Testbedingungen: 0,1 Mol/l Glycylglycin, pH 8,1
1,0 mMol/l Na-cholat
0,1 % Genapol X 080
0,12 mMol/l Nitroblautetrazoliumchlorid
1,5 mMol/l NAD
≧ 200 U/l Diaphorase (Cl. Kluyv.)
≧ 5000 U/l Glycerin-DH